(19) Europäisches Patentamt
European Patent Office
Office européen des brevets

(11) **EP 3 946 510 B1**

(12) **EUROPÄISCHE PATENTSCHRIFT**

(45) Veröffentlichungstag und Bekanntmachung des Hinweises auf die Patenterteilung:
**26.07.2023 Patentblatt 2023/30**

(21) Anmeldenummer: **20714954.3**

(22) Anmeldetag: **20.03.2020**

(51) Internationale Patentklassifikation (IPC):
*A61M 1/36* (2006.01)        *G01L 9/00* (2006.01)
*G01L 19/00* (2006.01)        *G01L 27/00* (2006.01)

(52) Gemeinsame Patentklassifikation (CPC):
**A61M 1/3641; G01L 9/0026; G01L 9/0089; G01L 19/0023; G01L 27/007;** A61M 2205/18

(86) Internationale Anmeldenummer:
**PCT/EP2020/057727**

(87) Internationale Veröffentlichungsnummer:
**WO 2020/193388 (01.10.2020 Gazette 2020/40)**

(54) **VERFAHREN UND VORRICHTUNG ZUR ÜBERWACHUNG DER MEMBRANKOPPLUNG BEI EINEM DRUCKMESSSYSTEM**

METHOD AND DEVICE FOR MONITORING MEMBRANE COUPLING IN A PRESSURE MEASURING SYSTEM

PROCÉDÉ ET DISPOSITIF POUR SURVEILLER L'ACCOUPLEMENT À DIAPHRAGME DANS UN SYSTÈME DE MESURE DE PRESSION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.03.2019 DE 102019107604**

(43) Veröffentlichungstag der Anmeldung:
**09.02.2022 Patentblatt 2022/06**

(73) Patentinhaber: **Fresenius Medical Care Deutschland GmbH
61352 Bad Homburg (DE)**

(72) Erfinder: **KOPPERSCHMIDT, Pascal
97456 Dittelbrunn (DE)**

(74) Vertreter: **Herrmann, Uwe
Lorenz Seidler Gossel
Rechtsanwälte Patentanwälte
Partnerschaft mbB
Widenmayerstraße 23
80538 München (DE)**

(56) Entgegenhaltungen:
**WO-A1-03/093780        WO-A1-2015/099932**

**Beschreibung**

Die vorliegende Erfindung betrifft ein Verfahren und eine Vorrichtung zur vorzugsweise kontinuierlichen Überwachung der Membrankopplung / Membranankopplung bei einem Druckmesssystem im extrakorporalen Kreislauf einer Blutbehandlungsmaschine.

Druckmessungen in einem extrakorporalen Kreislauf einer Blutbehandlungsmaschine erfolgen herkömmlicherweise entweder über lufthaltige Druckableitungsschläuche an den extrakorporalen Schlauchsegmenten unter Umständen mit integrierten Ausgleichsgefäßen oder Compliancegefäßen oder luftfrei direkt über eine im Schlauchsegment integrierte Membran, welche an einen Druckstempel (Druckdom) angekoppelt ist. Durch die Kopplung der Membran an den Druckdom kann eine zu einem bestimmten Druck proportionale Verformung der Membran mittels des Druckdoms gemessen werden und der entsprechende auf die Membran wirkende Druck ermittelt werden.

Die ermittelten Messwerte des Drucks, insbesondere des gegenüber der Atmosphäre negativen Vorlaufdruck im extrakorporalen Kreislauf, können zur Überwachung von rheologischen Blockaden, zur Korrektur von Flussraten peristaltischer Pumpen, zur Dichtigkeitsprüfung des Schlauchsystems, zur Überwachung druckerzeugender angeschlossener Aktoren und ggf. zur Erfassung von Vitalparametern des Patienten verwendet werden.

Aus dem Stand der Technik sind verschiedene Verfahren zur Druckmessung bekannt. Hierbei hat eine Druckmessung, welche über eine entlüftete Membran erfolgt, bestimmte Vorteile gegenüber einer Druckmessung über eine Druckleitung, welche mit Luft gefüllt ist.

Beispielsweise ist aus der WO 2015/099932 A1 bekannt, dass aufgrund der verminderten mechanischen Nachgiebigkeit (Compliance) der luftfreien Membran, mittels eines Druckmessverfahrens, welches eine luftfreie Membran einsetzt, Druckänderungen unmittelbar detektierbar sind. Im Gegensatz dazu erhöhen lufthaltige Druckableitungen das extrakorporale Blutvolumen, bedingen Luft-Blut Kontaktflächen, welche mit einer erhöhten Thrombosierungsgefahr verknüpft sind, und benötigen Protektoren, um ein Eindringen von Flüssigkeit in die Druckmesszelle zu vermeiden. Diese Protektoren können verstopfen und die Druckableitung unterbrechen.

Aber auch die Druckmessung unter Einsatz eines Druckdoms, wie sie herkömmlicherweise ausgeführt wird, ist problembehaftet. Beispielsweise ist aus der WO 03/093780 A1 bekannt, dass bei der Messung eines Drucks im Schlauch mit integriertem Druckdom unterhalb des Atmosphärendruckes die Kopplung zwischen Membran und Stempel, der die Membranposition erfasst, teilweise oder vollständig unterbrochen sein kann. Dieses Problem tritt insbesondere dann auf, wenn sich die Membran im Druckdom aufgrund des Unterdrucks nach innen (d.h. vom Druckdom weg in Richtung des Lumens eines Schlauchs, in dem die Membran installiert ist) wölbt und der Messstempel der Membran nur bedingt, d.h. nicht vollständig, folgen kann.

In diesem Fall tritt eine unerwünschte Entkopplung der Membran von dem Druckstempel bzw. Druckdom auf und die Messgenauigkeit des Druckmesssystems sinkt, da eine teilweise oder vollständige Lösung der Kopplung zu einer fehlerhaften Messung des Drucks im Schlauchsegment führt. Systeme, zu deren Überwachung oder Parametrisierung der Druck im Schlauch notwendig oder erforderlich ist, arbeiten unter diesen Bedingungen inkorrekt und können die Therapie oder den Patienten somit gefährden.

Um dieses Problem zu vermeiden wird herkömmlicherweise die Kopplung der Membran an den Druckdom überwacht, um so die Funktionsfähigkeit des Druckmesssystems zu überwachen. Die Überwachung der Kopplung zwischen Membran und Stempel erfolgt hierbei üblicherweise durch einen Drucktest, bei welchem der im Schlauchsystem vorliegende Druck kontrolliert vermindert wird und gleichzeitig der von dem Druckmesssystem ermittelte Wert auf Plausibilität überprüft wird.

Dieses herkömmliche Verfahren hat jedoch den signifikanten Nachteil, dass während der Ausführung des Verfahrens zur Überwachung der Membrankopplung einen gegebenenfalls mit der Blutbehandlungsmaschine, in deren extrakorporalem Kreislauf die Druckmessung erfolgt, durchgeführte extrakorporale Blutbehandlung unterbrochen werden muss.

In anderen Worten kann das herkömmliche Überwachungsverfahren nicht kontinuierlich bzw. bei fortlaufender Blutbehandlung ausgeführt werden. Folglich bietet das herkömmliche Verfahren lediglich eine Momentaufnahme der Ankopplung zwischen Membran und Druckdom des Druckmesssystems zum Zeitpunkt der Messung. Eine sich verändernde Kopplung zwischen der Membran und dem Druckdom während der Blutbehandlung kann durch dieses herkömmliche Überwachungsverfahren nicht erfasst werden.

Zur Sicherstellung der Patientensicherheit ist es jedoch unerlässlich, gerade während einer laufenden Blutbehandlung eine uneingeschränkte Funktionalität der eingesetzten Blutbehandlungsmaschine zu gewährleisten.

Der vorliegenden Erfindung liegt somit die Aufgabe zugrunde, ein Verfahren zu kontinuierlichen Überwachung der Membrankopplung in einem Druckmesssystem in einem extrakorporalen Kreislauf einer Blutbehandlungsmaschine bereitzustellen.

Diese Aufgabe wird durch ein Verfahren gemäß Anspruch 1 und eine Vorrichtung gemäß Anspruch 6 gelöst. Ein weiterer Aspekt der Erfindung betrifft eine Blutbehandlungsmaschine nach Anspruch 10. Weitere vorteilhafte Weiterbildungen der Erfindung sind Gegenstand der Unteransprüche.

Erfindungsgemäß umfasst ein Verfahren zur vorzugsweise kontinuierlichen Überwachung der Membrankopplung bei einem Druckmesssystem in einem extrakorporalen Kreislauf einer Blutbehandlungsmaschine die folgenden Schritte: Erfassung von im extrakorporalen Kreislauf auftretenden periodischen Druckpulsen; Frequenzanalyse auf der Grundlage der erfassten periodischen Druckpulse zur Berechnung von mindestens einem Fourierkoeffizienten; Vergleich des mindestens einen berechneten Fourierkoeffizienten hinsichtlich seiner Intensität mit mindestens einem zugehörigen Referenzwert; und Ausgeben eines Signals, wenn der Vergleich ergibt, dass der mindestens eine Fourierkoeffizient hinsichtlich seiner Intensität von dem zugeordneten Referenzwert über einen vorbestimmten Toleranzschwellenwert hinaus abweicht.

Der Kerngedanke der vorliegenden Erfindung besteht in anderen Worten darin, durch eine kontinuierliche Analyse des Messverhaltens des Druckmesssystems die Funktionalität des Druckmesssystems auch während des Betriebs des Druckmesssystems, z.B. während einer Blutbehandlung, kontinuierlich zu überwachen.

Das Signal kann beliebiger Art sein, es kann sich um ein internes, d.h. nach außen hin nicht wahrnehmbares Signal handeln oder um ein externe Signal, d.h. um ein Signal, das durch einen Nutzer wahrnehmbar ist, wie z.B. um ein optisches und/akkustisches Alarmsignal. Das genannte interne Signal können beispielsweise Daten sein, die eine bestimmte Maßnahme auslösen, wie z.B. eine Fehlerroutine triggern.

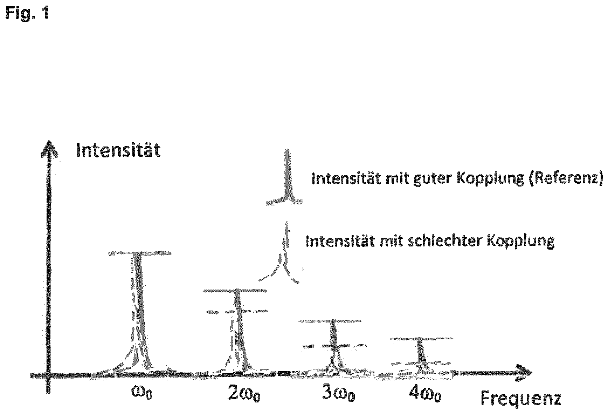

Eine Fehlfunktion des Druckmesssystems, insbesondere in Form einer mangelhaften oder nur ungenügenden Kopplung zwischen der Membran und dem Druckdom / Druckstempel des Druckmesssystems spiegelt sich insbesondere in einer verringerten Intensität der Fourierkoeffizienten einer den periodisch gemessenen Druckpulsen zugrunde liegenden Fourierreihe mit zunehmender Frequenz wieder. In anderen Worten ist es charakteristisch für ein Druckmesssystem mit ungenügender Membrankopplung, dass niederfrequente, harmonisch von niederer Ordnung seiende Druckpulse noch angemessen erfasst werden können, jedoch bei höherfrequenten, harmonisch von höherer Ordnung seienden Druckpulsen die durch die Entkoppelung zwischen Membran und Druckdom / Druckstempel gedämpfte bzw. verschlechterte Signalübertragung zu einer verschlechterten Signalerfassung, und somit zu Fourierkoeffizienten von verringerter Intensität, führt. Dieses Prinzip ist in Fig. 1 bildlich dargestellt.

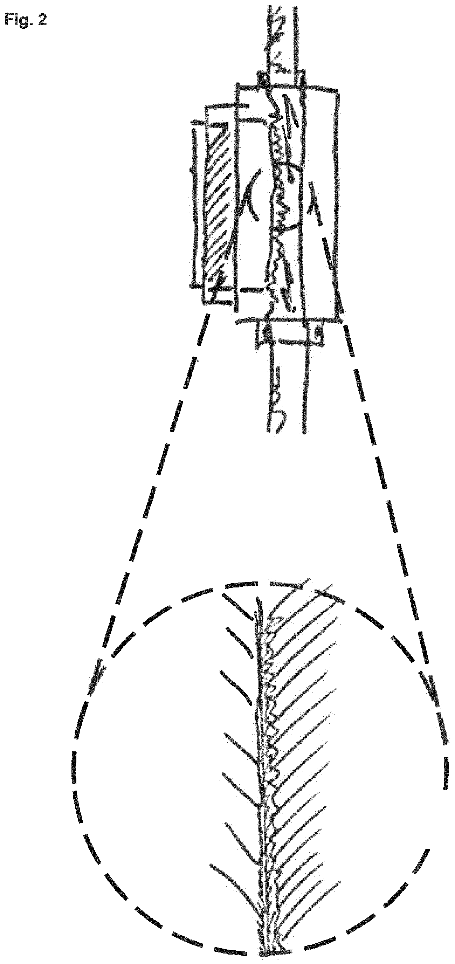

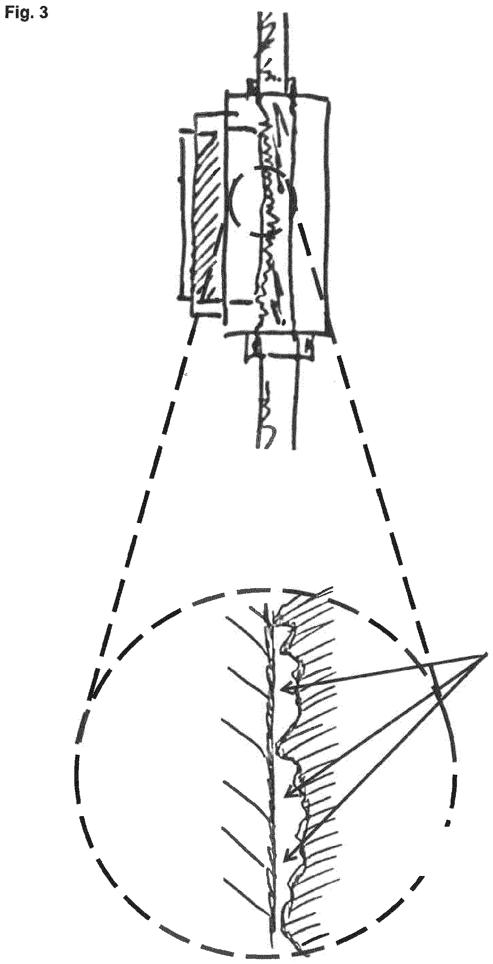

Zudem verdeutlichen die Figuren 2 und 3 den Zustand eines Druckmesssystems bei guter Kopplung der Membran an den Messstempel des Druckmesssystems (Fig. 2) sowie bei mangelhafter Kopplung der Membran an den Messstempel des Druckmesssystems (Fig. 3).

Wie in Fig. 1 gezeigt, ist ein Druckmesssystem 1 an einer Schlauchleitung 2, welche Teil eines extrakoporalen Kreislaufs einer Blutbehandlungsmaschine sein kann, angeordnet. Vorzugsweise bildet das Druckmesssystem einen Teil des extrakoporalen Kreislaufs. Das Druckmesssystem 1 weist einen Druckdom / Messstempel 3 und eine Membran 4 auf.

Wie in der Detailansicht in Fig. 2 gezeigt, liegt bei optimaler oder auch nur ausreichender Kopplung der Membran 4 an den Messstempel 3 die Membran 4 an dem Messstempel 3 an und bildet somit eine vorzugsweise durchgängige Kontaktfläche 5 zwischen der Membran 4 und dem Messstempel 3 aus.

Bei einer derartigen Kopplung der Membran 4 an den Messstempel 3 werden druckbedingte Verformungen der Membran 4 unmittelbar an den Messstempel 3 übertragen, sodass die Spezifität und Messgenauigkeit des Druckmesssystems gut oder zufriendenstellend ist.

Wie in Fig. 3 gezeigt, befindet sich bei mangelhafter Kopplung der Membran 4 an den Messstempel 3 des Druckmesssystems 1 die Mebran 4 nicht in Anlage an dem Messstempel 3.

Es wird somit keine durchgängige Kontaktfläche 5 zwischen der Membran 4 und dem Messstempel 3 ausgebildet, vielmehr existieren Kavitäten 6 und / oder Gasblasen zwischen der Membran 4 und dem Messstempel 3. Die Messgenauigkeit des Druckmesssystems ist in einem derartigen Zustand aufgrund der gedämpften bzw. abgeschwächten Signalübertragung von der Membran 4 zu dem Messstempel 3 verringert. Dieser Effekt schlägt sich insbesondere bei höherfrequenten Signalen nieder.

Es hat sich als vorteilhaft erwiesen, wenn bei einem erfindungsgemäßen Verfahren bei dem Vergleich die absolute oder relative Änderung des mindestens einen berechneten Fourierkoeffizienten hinsichtlich seiner Intensität bezüglich des mindestens einen zugehörigen Referenzwerts ermittelt wird. Der mindestens eine Referenz wert oder auch mehrere referenzwerte sind vorzugsweise im Vorhinein abgelegt bzw. abgespeichert worden.

Alternativ oder zusätzlich kann bei dem Vergleich auch eine Änderung des Zeitverlaufs oder Trends des mindestens einen berechneten Fourierkoeffizienten hinsichtlich seiner Intensität bezüglich einer vorbestimmten zeitlichen Änderung ermittelt wird. In anderen Worten muss der Referenzwert nicht zwingend ein absoluter Wert sein, sondern kann auch eine bestimmte Änderungsrate oder eine sonstige Vergleichsgröße sein.

Vorzugsweise wird ein Signal ausgeben, wenn der Vergleich ergibt, dass die Intensität des mindestens einen Fourierkoeffizienten mit zunehmender Frequenz der im extrakorporalen Kreislauf auftretenden periodischen Druckpulse abnimmt.

Weiterhin hat es sich als vorteilhaft erwiesen, wenn eine Mehrzahl an Fourierkoeffizienten vorzugsweise mehrerer höherer harmonischer Fourierreihen berechnet wird, welche jeweils mit einem zugeordneten Referenzwert verglichen werden. Die Verwendung von Koeffizienten mehrerer höherer harmonischer Fourierreihen ermöglicht ein redundantes Verfahren mit erhöhter Spezifität.

Weiterhin kann zusätzlich vorgesehen sein, dass ein vorzugsweise vollautomatischer Reparaturversuch zur Verbesserung der Membrankopplung oder Wiederher-Weiterhin kann zusätzlich vorgesehen sein, dass mittels einer erfindungsgemäßen Vorrichtung ein vorzugsweise vollautomatischer Reparaturversuch zur Verbesserung der Membrankopplung oder Wiederherstellung einer angemessenen Membrankopplung bei dem Druckmesssystem vorzugsweise selbstständig / selbsttätig ausgeführt wird, wenn der Vergleich ergibt, dass der mindestens eine Fourierkoeffizient hinsichtlich seiner Intensität von dem zugeordneten Referenzwert über einen vorbestimmten Toleranzschwellenwert hinaus abweicht.

In anderen Worten beschränkt sich das erfindungsgemäße Verfahren, wie es von einer erfindungsgemäßen Vorrichtung ausgeführt wird, nicht zwangsläufig auf das Ausgeben eines Signals bei der Erfassung einer Fehlfunktion des Druckmesssystems, sondern initiiert gegebenenfalls einen Versuch zur Behebung der Fehlfunktion und / oder Wiederherstellung der Funktionalität des Druckmesssystems bzw. zur Reparatur des Druckmesssystems.

Der Reparaturversuch kann den folgenden Schritte umfassen: Anlegen eines positiven Drucks im extrakorporalen Kreislauf, derart, dass sich die Membran des Druckmesssystems hin zu dem Druckdom des Druckmesssystems wölbt, wodurch etwaige Gasblasen von einer Kontaktfläche zwischen der Membran und dem Druckdom oder Druckstempel entfernt werden. Durch die Entfernung der Gasblasen wird die Kopplung zwischen der Membran und dem Druckdom / Druckstempel verbessert.

Weiterhin betrifft die Erfindung eine Vorrichtung zur Ausführung eines erfindungsgemäßen Verfahrens mit einem Druckmesssystem, welches einen Druckdom und eine an den Druckdom gekoppelte Membran aufweist, und einer Steuerungseinheit, welche dazu ausgelegt ist, die Verfahrensschritte eines erfindungsgemäßen Verfahrens auszuführen. Zudem kann die Vorrichtung einen Speicher aufweisen, im welchem die Referenzwerte abgelegt bzw. abgespeichert sind.

Die Blutbehandlungsmaschine weist hierbei vorzugsweise mindestens eine vorzugsweise peristaltische Pumpe auf, welche mit einer bestimmten Frequenz stoßweise ein Fluidvolumen fördert. Die Blutbehandlungsmaschine kann aber auch einen beliebigen anderen Aktor aufweisen, welcher periodisch wiederkehrende Druckpulse im extrakorporalen Kreislauf der Blutbehandlungsmaschine bewirkt.

Die vorliegende Erfindung beruht grundsätzlich auf den folgenden Überlegungen:
Die Förderung von Volumen in extrakorporale Schlauchsysteme oder einen extrakorporalen Kreislauf einer Blutbehandlungsmaschine erfolgt in der Regel über nichtinvasive peristaltische Pumpen, welche ein im Pumpschlauchsegment eingeschlossene Volumen durch eine peristaltische Bewegung stromab bewegen.

Diese Bewegung ist aufgrund der periodisch eingreifenden Pumpköpfe auf das Schlauchsegment diskontinuierlich. In anderen Worten wird das Fördervolumen einer peristaltischen Pumpe stoßweise gefördert. Miteinhergehend mit dieser diskontinuierlichen Förderung sind periodische Druckschwankungen, welche sich stromab und stromauf der Pumpe ausbreiten. Die Periodizität der Druckschwankung leitet sich aus der Rotationsgeschwindigkeit der Pumpe und somit der Häufigkeit bzw. der Frequenz der geförderten Stöße an Fördervolumen der Pumpe ab.

Diese Druckschwankungen lassen sich mittels der im extrakorporalen Schlauch angeordneten bzw. angekoppelten Druckmesssysteme (Drucksensoren) detektieren. Geeignete Algorithmen ermöglichen hierbei eine Analyse der Druckwerte z.B. hinsichtlich ihrer Fourierkoeffizienten.

Insbesondere die Fourierkoeffizienten eignen sich zur Beschreibung bzw. Analyse der Periodizität der periodisch auftretenden Druckschwankungen auf der Grundlage der Messwerte der Druckmesssysteme. Die Fourierkoeffizienten stellen den Beitrag zu dem Gesamtsignal einer einzelnen harmonischen Schwingung einer bestimmten Periodizität dar. Ändert sich ein Koeffizient, so hat sich der Beitrag dieser entsprechenden Periodizität im Gesamtsignal geändert.

Die Frequenzanalyse der Druckpulse (bzw. der in Erwiderung auf die periodischen Druckschwankungen ermittelten Druckmesswerte) und die Bestimmung der jeweiligen frequenzspezifischen Beiträge zum periodischen Signal liefern Informationen zur veränderlichen Güte der Ankopplung zwischen der Membran und dem Druckdom bzw. Druckstempel.

Aufgrund der üblichen Formstabilität der Druckpulse (für eine jede vorbestimmte Einstellung der peristaltischen Pumpe existiert ein im Wesentlichen unveränderliches definiertes Muster an periodischen Druckschwankungen und somit an Druckmesswerten bzw. Druckpulsen) ist eine Änderung der frequenzspezifischen Beiträge zu dem Drucksignal sehr wahrscheinlich auf eine Minderung der Membrankopplung im Druckdom zurückzuführen.

In anderen Worten deutet eine Veränderung der Messwerte, welche insbesondere durch die Analyse der Fourierkoeffizienten erfasst wird, in Anbetracht des unveränderlichen definierten Musters periodischer Druckschwankungen aufgrund des Betriebes der peristaltischen Pumpe auf eine Veränderung des Druckmesssystems selbst, insbesondere auf eine Veränderung der Kopplung zwischen Membran und Drucksensor, hin.

Insbesondere ist bei einer Abnahme der Membrankopplung eine Abschwächung der Fourierkoeffizienten der höheren Frequenzen zu erwarten, siehe Fig. 1. In anderen Worten werden bei einer Abnahme der Kopplung der Membran an den Druckdom die Fourierkoeffizienten bei zunehmender Frequenzhöhe schwächer.

Dies ist eine Folge einer gedämpften bzw. weniger unmittelbaren Übertragung von Signalen mit höher-frequenter Feinstruktur, wenn die Membran nicht, wie dies eigentlich idealerweise der Fall sein sollte, unmittelbar an den Druckdom gekoppelt ist.

Dieser Effekt kann dazu genutzt werden, die Kopplung zwischen Membran und Dom kontinuierlich zu überwachen, wobei eine absolute und / oder eine relative (z.B. relativ zu bestimmten Referenzwerten) Änderung der Beiträge der Koeffizienten auf eine Änderung der Kopplung hinweist.

Im Folgenden wird die Erfindung unter Bezugnahme auf eine Ausführungsform der Erfindung weiter erläutert.

Bei der vorliegenden Ausführungsform wird ein erfindungsgemäßes Verfahren dazu eingesetzt, die Funktion eines Druckmesssystems, insbesondere die Kopplung einer Membran an einen Druckdom des Druckmesssystems, in einem extrakorporalen Kreislauf einer Blutbehandlungsmaschine zu überwachen.

Um die Kopplung zwischen der Membran am Schlauchsegment und einem Messstempel am Druckdom kontinuierlich zu überwachen, werden die von der Blutpumpe oder anderen Pumpen im extrakorporalen Kreislauf ausgehenden Druckschwankungen mittels des Druckmesssystems bzw. Drucksensors erfasst und in einer geeigneten Recheneinheit vorzugsweise mittels Fourieranalyse spektral in Komponenten bzw. Fourierkoeffizienten zerlegt.

Diese Komponenten bzw. Fourierkoeffizienten werden hinsichtlich ihrer Intensität mit Referenzwerten verglichen. Weichen die Intensitäten um einen bestimmten, vorzugsweise im Vorhinein definierten, Teil oder Betrag von dem jeweiligen Referenzwert ab, so wird erfasst, dass die Übertragung des Drucksignals suboptimal bzw. geschwächt ist und vorzugsweise wird ein entsprechendes Signal bzw. eine entsprechende Meldung ausgegeben.

Mittels eines automatischen oder manuellen Plausibilitätchecks kann überprüft bzw. ermittelt werden, welche die mögliche Ursache für die Schwächung der Signalübertragung ist. Beispielsweise kann die Signalübertragung aufgrund einer mangelhaften Kopplung der Membran an den Druckdom auftreten.

Der Vergleich der Intensitäten der Komponenten bzw. Fourierkoeffizienten mit entsprechenden Referenzwerten erfolgt vorzugsweise wie nachstehend dargelegt. Vorzugsweise ist einer jeden berechneten Intensität $A_i$ ein entsprechender Referenzwert Ri zugeordnet, sodass eine absolute oder relative Änderung zur Referenz (crit$_i$) festgestellt werden kann (siehe Gleichung 1).

$$\left| \frac{\partial}{\partial t} A_i \right| > crit_i$$

Gleichung 1

Alternativ oder zusätzlich ist es auch denkbar, den Trend bzw. Zeitverlauf einer bestimmten Intensität zu überwachen. In diesem Fall sind Intensitäts-Referenzwerte nicht zwingen erforderlich. Beispielsweise kann Überwacht werden, ob die Abweichung zwischen einer Intensität $A_i$ und einem entsprechenden Referenzwert R*i* über einen bestimmten Referenzwert bzw. einen Schwellenwert crit$_i$ hinausgeht. Außerdem kann es auch vorgesehen sein, dass der Trendverlauf der berechneten Intensität eine bestimmte zeitliche Änderung nicht über- bzw. unterschreiten darf, um einen Fehlerfall zu signalisieren (siehe Gleichung 2).

$$A_i - R_i < crit_i \; ; \; \frac{A_i - R_i}{R_i} < crit_i$$

Gleichung 2

Überschreitet die Änderungsrate des Zeitverlaufs einer Intensität beispielsweise eine bestimmte vordefinierte Änderungsrate oder verlässt die Änderungsrate des Zeitverlaufs einen vorbestimmten Toleranzkorridor, so wird vorzugsweise ein Signal ausgegeben.

Gegebenenfalls kann bei einer Identifizierung einer gestörten Signalübertragung das Druckmesssystem automatisch einen Reparaturversuch unternehmen, um das Problem zu beseitigen.

Im Falle des mangelhafte angekoppelten Druckdomes kann dieser bei leicht positiven Druck im Schlauch die Membran kurzzeitig freigeben, sodass aufgrund der zum Druckdom hin gewölbte Membran mögliche Luftblasen, welche zur Minderung der Kopplung beitragen, aus der Kontaktfläche zwischen Membran und Druckdom herausgeschoben werden.

Das erfindungsgemäße Überwachungsverfahren bietet mindestens die nachstehenden Vorteile:
Das Verfahren kann kontinuierliche betrieben werden. Eine etwaige Blutbehandlung muss somit nicht unterbrochen werden.

Die Methode der Verwendung von Koeffizienten mehrerer höherer harmonischer Fourierreihen ermöglicht ein redundantes Verfahren mit erhöhter Spezifität.

Die Lösung der Membrankopplung kann graduell erfasst werden und mit einem hinterlegten Referenzwert verglichen werden.

Infolge der ermittelten Abweichung zwischen einem aktuellen Messwert bzw. einem aus diesem berechneten Fourierkoeffizienten und einem Referenzwert können weitere Prüfungen oder Methoden vorzugsweise automatisch initiiert werden, um die Membrankopplung zu verbessern.

Alternativ oder zusätzlich kann auch ein Signal in Form eines Alarmsignals ausgegeben werden, welches einen Benutzer auf eine wahrscheinliche Fehlfunktion des Druckmesssystems hinweist und ihn oder sie anregt, das zugrundeliegende Problem zu beheben.

Aufgrund der Vielzahl von höheren harmonischen Fourierreihen, die typischerweise in extrakorporalen Systemen mit druckpulserzeugenden Aktoren, wie beispielsweise peristaltischen Pumpen, aufgrund der reduzierten mechanischen Nachgiebigkeit bzw. Compliance vorhanden sind, lassen sich die einzelnen Intensitäten der höheren harmonischen Fourierreihen oder Gruppen von Intensitäten einander gegenüberstellen bzw. jeweils mit Referenzwerten vergleichen. Dies erweitert die Anwendungsmöglichkeiten des erfindungsgemäßen Verfahrens und erhöht die Aussagekraft und Sensitivität der Analyse.

Das Verfahren kann unter Verwendung beliebiger Aktoren, beispielsweise Pumpen, angewendet werden, deren Aktivität zur Erzeugung und Ausbreitung von periodischen Druckpulsen führt. Selbst nicht-peristaltisch arbeitende Pumpen können verwendet werden, wenn deren Förderrate mit einer bestimmten Frequenz moduliert wird.

Denkbar ist es, dass im Rahmen einer Fehlerroutine, die aufgrund der Vorgehensweise in Anspruch 1 ausgelöst wird, d.h. wenn er Vergleich ergibt, dass der mindestens eine Fourierkoeffizient hinsichtlich seiner Intensität von dem zugeordneten Referenzwert über einen vorbestimmten Toleranzschwellenwert hinaus abweicht, untersucht wird, ob ein Membrankopplungsproblem oder ein sonstiger Fehler vorliegt.

Neben der kontinuierlichen Überwachung der Kopplung der Membran an den Druckdom lassen sich darüber hinaus Knickstellen zwischen dem die Druckpulse generierenden Aktor (z.B. Pumpe) und der im Schlauch befindlichen Messstelle des Druckmesssystems identifizieren.

Diese Knickstellen, welche z.B. aufgrund einer Torsion eines Schlauchsegments zwischen Sensor und Aktor auftreten, führen zu einer Dämpfung der Signalübertragung, welches sich mathematisch als Abnahme der Intensitätswerte von Fourierkoeffizienten bei höheren Frequenzen darstellen lässt.

Die kontinuierliche Überprüfung einer hinreichenden Ankopplung einer Membran an einen Druckdom kann weiterhin im Allgemeinen auf andere mechanisch zu koppelnde Systeme erweitert werden.

Hierzu zählen z.B. Membranen zur Schallübertragung oder Folien zum Einschließen von Kavitäten oder Hohlräumen. Eine Anwendung des vorliegenden erfindungsgemäßen Verfahrens auf derartige Systeme ist von der vorliegenden Erfindung mitumfasst.

## Patentansprüche

1. Verfahren zur vorzugsweise kontinuierlichen Überwachung der Membrankopplung bei einem Druckmesssystem in einem extrakorporalen Kreislauf einer Blutbehandlungsmaschine, mit den Schritten:

   - Erfassung von im extrakorporalen Kreislauf auftretenden periodischen Druckpulsen;
   - Frequenzanalyse auf der Grundlage der erfassten periodischen Druckpulse zur Berechnung von mindestens einem Fourierkoeffizienten;
   - Vergleich des mindestens einen berechneten Fourierkoeffizienten hinsichtlich seiner Intensität mit mindestens einem zugehörigen Referenzwert; und
   - Ausgeben eines Signals, wenn der Vergleich ergibt, dass der mindestens eine Fourierkoeffizient hinsichtlich seiner Intensität von dem zugeordneten Referenzwert über einen vorbestimmten Toleranzschwellenwert hinaus abweicht.

2. Verfahren nach Anspruch 1, **dadurch gekennzeichnet, dass** bei dem Vergleich die absolute oder relative Änderung oder Abweichung des mindestens einen berechneten Fourierkoeffizienten hinsichtlich seiner Intensität bezüglich des mindestens einen zugehörigen Referenzwerts ermittelt wird und/oder dass es sich bei dem Signal um ein wahrnehmbares Signal handelt, wie ein akustisches und/oder optisches Alarmsignal, oder um ein nicht wahrnehmbares Signal, wie ein Signal zum Auslösen einer Fehlerroutine.

3. Verfahren nach einem der Ansprüche 1 oder 2, **dadurch gekennzeichnet, dass** bei dem Vergleich eine Änderung des Zeitverlaufs oder Trends des mindestens einen berechneten Fourierkoeffizienten hinsichtlich seiner Intensität bezüglich einer vorbestimmten zeitlichen Änderung ermittelt wird.

4. Verfahren nach einem der vorhergehenden Ansprüche, **dadurch gekennzeichnet, dass** das Signal ausgeben wird, wenn der Vergleich ergibt, dass die Intensität des mindestens einen Fourierkoeffizienten mit zunehmender Frequenz der im extrakorporalen Kreislauf auftretenden periodischen Druckpulse abnimmt.

5. Verfahren nach einem der vorhergehenden Ansprüche, **dadurch gekennzeichnet, dass** eine Mehrzahl an Fourierkoeffizienten vorzugsweise mehrerer höherer harmonischer Fourierreihen berechnet wird, welche jeweils mit einem zugeordneten Referenzwert verglichen werden.

6. Vorrichtung zur Ausführung eines Verfahrens nach einem der Ansprüche 1 bis 5, mit einem Druckmesssystem, welches einen Druckdom und eine an den Druckdom gekoppelte Membran aufweist, und einer Steuerungseinheit, welche dazu ausgelegt ist, die Verfahrensschritte gemäß einem der Ansprüche 1 bis 5 auszuführen.

7. Vorrichtung nach Anspruch 6, **dadurch gekennzeichnet, dass** das Druckmesssystem dazu ausgelegt und / oder angeordnet ist, den Druck in einem extrakorporalen Kreislauf einer Blutbehandlungsmaschine zu messen.

8. Vorrichtung gemäß Anspruch 6, wobei die Steuerungseinheit weiter dazu ausgelegt ist, eine vorzugsweise selbstständige Ausführung eines vorzugsweise vollautomatischen Reparaturversuchs als Bestandteil einer Fehlerroutine zur Verbesserung der Membrankopplung oder Wiederherstellung einer angemessenen Membrankopplung bei dem Druckmesssystem durchzuführen, wenn der Vergleich ergibt, dass der mindestens eine Fourierkoeffizient hinsichtlich seiner Intensität von dem zugeordneten Referenzwert über einen vorbestimmten Toleranzschwellenwert hinaus abweicht.

9. Vorrichtung gemäß Anspruch 8, **dadurch gekennzeichnet, dass** der Reparaturversuch die folgenden Schritte umfasst:

- Anlegen eines positiven Drucks im extrakorporalen Kreislauf, derart, dass sich die Membran des Druckmesssystems hin zu dem Druckdom des Druckmesssystems wölbt, wodurch etwaige Gasblasen von einer Kontaktfläche zwischen der Membran und dem Druckdom oder Druckstempel entfernt werden.

10. Blutbehandlungsmaschine mit einer Vorrichtung nach einem der Ansprüche 6 bis 9 .

11. Blutbehandlungsmaschine nach Anspruch 10, **dadurch gekennzeichnet, dass** die Blutbehandlungsmaschine mindestens eine vorzugsweise peristaltische Pumpe aufweist, welche mit einer bestimmten Frequenz stoßweise ein Fluidvolumen fördert.

**Claims**

1. A method for monitoring, preferably continuously monitoring, the membrane coupling in a pressure measurement system in an extracorporeal circuit of a blood treatment machine comprising the steps:

- detecting periodic pressure pulses occurring in the extracorporeal circuit;
- a frequency analysis based on the detected periodic pressure pulses for calculating at least one Fourier coefficient;
- comparing the at least one calculated Fourier coefficient with respect to its intensity with at least one associated reference value; and
- outputting a signal when the comparison shows that, with respect to its intensity, the at least one Fourier coefficient differs from the associated reference value beyond a predetermined tolerance threshold value.

2. A method in accordance with claim 1, **characterized in that**, on the comparison, the absolute or relative change or deviation of the at least one calculated Fourier coefficient is determined with respect to its intensity with regard to the at least one associated reference value; and/or **in that** the signal is a perceptible signal such as an acoustic and/or optical alarm signal or an imperceptible signal such as a signal to trigger an error routine.

3. A method in accordance with one of the claims 1 or 2, **characterized in that**, on the comparison, a change of the time progression or trend of the at least one calculated Fourier coefficient is determined with respect to its intensity with regard to a predetermined time change.

4. A method in accordance with one of the preceding claims, **characterized in that** the signal is output when the comparison shows that the intensity of the at least one Fourier coefficient reduces as the frequency of the periodic pressure pulses occurring in the extracorporeal circuit increases.

5. A method in accordance with one of the preceding claims, **characterized in that** a plurality of Fourier coefficients of preferably a plurality of higher harmonic Fourier series are calculated that are each compared with an associated reference value.

6. An apparatus for carrying out a method in accordance with one of the claims 1 to 5, having a pressure measurement system that has a pressure dome and a membrane coupled to the pressure dome and having a control unit that is configured to carry out the method steps in accordance with one of the claims 1 to 5.

7. An apparatus in accordance with claim 6, **characterized in that** the pressure measurement system is configured and/or arranged to measure the pressure in an extracorporeal circuit of a blood treatment machine.

8. An apparatus in accordance with claim 6, wherein the control unit is further configured to preferably independently carry out a repair attempt, preferably a fully automatic repair attempt, as an element of an error routine to improve the membrane coupling or to restore an appropriate membrane coupling in the pressure measurement system when the comparison shows that the at least one Fourier coefficient differs from the associated reference value with respect to its intensity beyond a predetermined tolerance threshold value.

9. An apparatus in accordance with claim 8, **characterized in that** the repair attempt comprises the following steps:

   - Applying a positive pressure in the extracorporeal circuit such that the membrane of the pressure measurement system arches toward the pressure dome of the pressure measurement system, whereby any gas bubbles are removed from a contact surface between the membrane and the pressure dome or the pressure plunger.

10. A blood treatment machine having an apparatus in accordance with one of the claims 6 to 9.

11. A blood treatment machine in accordance with claim 10, **characterized in that** the blood treatment machine has at least one pump, preferably a peristaltic pump, that intermittently conveys a fluid volume at a specific frequency.

**Revendications**

1. Procédé de surveillance, de préférence continue, de l'accouplement de la membrane dans un système de mesure de pression dans un circuit extracorporel d'une machine de traitement du sang, avec les étapes suivantes :

   - la détection d'impulsions de pression périodiques se produisant dans le circuit extracorporel ;
   - l'analyse de fréquence sur la base des impulsions de pression périodiques détectées pour calculer au moins un coefficient de Fourier ;
   - la comparaison de l'au moins un coefficient de Fourier calculé en ce qui concerne son intensité avec au moins une valeur de référence associée ; et
   - l'émission d'un signal lorsque la comparaison montre que l'au moins un coefficient de Fourier s'écarte en ce qui concerne son intensité de la valeur de référence associée au-delà d'une valeur seuil de tolérance prédéterminée.

2. Procédé selon la revendication 1, **caractérisé en ce que**, lors de la comparaison, la variation ou l'écart absolu ou relatif de l'au moins un coefficient de Fourier calculé en ce qui concerne son intensité par rapport à l'au moins une valeur de référence associée est déterminé et/ou **en ce que** le signal consiste en un signal perceptible, tel qu'un signal d'alarme acoustique et/ou optique, ou en un signal non perceptible, tel qu'un signal de déclenchement d'un sous-programme d'erreur.

3. Procédé selon l'une quelconque des revendications 1 ou 2, **caractérisé en ce que**, lors de la comparaison, une variation de l'évolution temporelle ou de la tendance de l'au moins un coefficient de Fourier calculé est déterminée en ce qui concerne son intensité par rapport à une variation temporelle prédéterminée.

4. Procédé selon l'une quelconque des revendications précédentes, **caractérisé en ce que** le signal est émis lorsque la comparaison montre que l'intensité de l'au moins un coefficient de Fourier diminue lorsque la fréquence des impulsions de pression périodiques se produisant dans le circuit extracorporel augmente.

5. Procédé selon l'une quelconque des revendications précédentes, **caractérisé en ce qu'**une pluralité de coefficients de Fourier, de préférence plusieurs séries de Fourier harmoniques supérieures, sont calculés, qui sont respectivement comparés à une valeur de référence associée.

6. Dispositif pour l'exécution d'un procédé selon l'une quelconque des revendications 1 à 5, avec un système de mesure de pression qui présente un dôme de pression et une membrane accouplée au dôme de pression, et une unité de commande qui est conçue pour exécuter les étapes de procédé selon l'une quelconque des revendications 1 à 5.

7. Dispositif selon la revendication 6, **caractérisé en ce que** le système de mesure de pression est conçu et/ou agencé pour mesurer la pression dans un circuit extracorporel d'une machine de traitement du sang.

8. Dispositif selon la revendication 6, dans lequel l'unité de commande est en outre conçue pour effectuer une exécution, de préférence automatique, d'un essai de réparation, de préférence entièrement automatique, dans le cadre d'un sous-programme d'erreur pour améliorer l'accouplement de la membrane ou pour rétablir un accouplement de la membrane mesuré dans le système de mesure de pression lorsque la comparaison montre que l'au moins un coefficient de Fourier s'écarte, en ce qui concerne son intensité, de la valeur de référence associée, au-delà d'un seuil de tolérance prédéterminé.

9. Dispositif selon la revendication 8, **caractérisé en ce que** l'essai de réparation comprend les étapes suivantes :

- l'application d'une pression positive dans le circuit extracorporel de telle sorte que la membrane du système de mesure de pression se bombe vers le dôme de pression du système de mesure de la pression, ce qui permet d'éliminer toute bulle de gaz d'une surface de contact entre la membrane et le dôme de pression ou le tampon de pression.

10. Machine de traitement du sang avec un dispositif selon l'une quelconque des revendications 6 à 9.

11. Machine de traitement du sang selon la revendication 10, **caractérisée en ce que** la machine de traitement du sang présente au moins une pompe, de préférence péristaltique, qui refoule un volume de fluide par à-coups à une fréquence déterminée.

Fig. 1

**Fig. 2**

**Fig. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2015099932 A1
- WO 03093780 A1